# FASCICULE DE BREVET EUROPEEN

(11) **EP 1 299 072 B1**
(45) Date de publication et mention de la délivrance du brevet: **16.11.2005**
(21) Numéro de dépôt: 01949592.8
(22) Date de dépôt: 29.06.2001
(51) Int. Cl.: A61K 7/13

(54) **COMPOSITION DE TEINTURE D'OXYDATION POUR FIBRES KERATINIQUES COMPRENANT UN POLYMERE AMPHOTERE A CHAINE GRASSE**
ZUSAMMENSETZUNG ZUR OXIDATIVEN FÄRBUNG VON KERATINFASERN, DIE EIN ANIONISCHES AMPHIPHILES, MIT EINER FETTKETTE MODIFIZIERTES POLYMER ENTHÄLT
OXIDATION DYEING COMPOSITION FOR KERATINOUS FIBRES COMPRISING AN AMPHOTERIC POLYMER WITH FATTY CHAIN

(30) Priorité: 30.06.2000 FR 0008503
(43) Date de publication de la demande: 09.04.2003
(73) Titulaire: L'OREAL, 75008 Paris (FR)
(72) Inventeur: LAURENT, Florence, 92270 BOIS COLOMBES (FR)
(74) Mandataire: Wattremez, Catherine
(86) Numéro de dépôt international: PCT/FR2001/002095
(87) Numéro de publication internationale: WO 2002/000181

(56) Documents cités:
- EP-A- 1 062 935
- EP-A- 1 064 915
- EP-A- 1 064 916
- EP-A- 1 064 917
- EP-A- 1 064 927
- EP-A- 1 068 857
- WO-A-00/39176
- DE-C- 4 404 493
- US-A- 3 860 699
- US-A- 4 402 977
- US-A- 4 814 101
- US-A- 4 938 950
- US-A- 5 879 670

## Description

La présente invention concerne une composition pour la teinture d'oxydation des fibres kératiniques et en particulier des fibres kératiniques humaines telles que les cheveux, comprenant au moins un précurseur de colorant d'oxydation et/ou un coupleur particulier et au moins un polymère amphotère comportant au moins une chaîne grasse.

Il est connu de teindre les fibres kératiniques et en particulier les cheveux humains, avec des compositions de teinture contenant des colorants d'oxydation et notamment des précurseurs de colorants d'oxydation, généralement appelés "bases d'oxydation", en particulier des ortho- ou para- phénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des «bases d'oxydation» sur elles-mêmes, soit d'une condensation oxydative des «bases d'oxydation» sur d'autres colorants d'oxydation qui sont des composés modificateurs de coloration, ou «coupleurs», qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les «bases d'oxydation» et d'autre part par les «coupleurs», permet l'obtention d'une palette très riche en coloris.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Pour localiser le produit de coloration d'oxydation à l'application sur les cheveux afin qu'il ne coule pas sur le visage ou en dehors des zones que l'on se propose de teindre, outre l'emploi d'épaississants traditionnels tels que l'acide polyacrylique réticulé, les hydroxyéthylcelluloses, les cires, et des mélanges d'agents tensio-actifs non-ioniques de HLB (Hydrophilic Lipophilic Balance), convenablement choisis, on a aussi eu recours à des polymères associatifs de type anionique, non-ionique ou cationique.

Cependant, les systèmes épaississants traditionnels, les cires et lesdits mélanges de tensio-actifs mentionnés ci-dessus ne permettent pas d'obtenir des nuances puissantes et chromatiques de faibles sélectivités et de bonnes ténacités tout en assurant un bon état cosmétique à la chevelure traitée. Par ailleurs, les compositions tinctoriales prêtes à l'emploi contenant le ou les colorants d'oxydation et lesdits systèmes épaississants de l'art antérieur ne permettent pas une application suffisamment précise sans coulures ni chutes de viscosité dans le temps.

Les polymères associatifs de type anionique, non-ionique ou cationique représentent un progrès important dans la recherche d'une solution à ce problème.

Voici maintenant que la demanderesse a découvert, de façon tout à fait inattendue et surprenante, de nouvelles compositions de teinture d'oxydation (après mélange avec l'oxydant) qui ne coulent pas et restent donc mieux localisées au point d'application et qui permettent d'obtenir des nuances puissantes et chromatiques de faibles sélectivités et de bonnes ténacités tout en assurant un bon état cosmétique à la chevelure traitée, lesdites compositions de teinture comprenant une quantité efficace d'un polymère amphotère comportant au moins une chaîne grasse que l'on introduit (i) soit dans la composition contenant le ou les précurseurs de colorant d'oxydation et/ou coupleurs particuliers, soit (ii) dans la composition oxydante, ou (iii) dans les deux compositions à la fois.

Cette découverte est à la base de la présente invention.

La présente invention a ainsi pour objet une nouvelle composition pour la teinture d'oxydation des fibres kératiniques, en particulier des fibres kératiniques humaines, telles que les cheveux, comprenant, dans un milieu approprié pour la teinture, au moins un précurseur de colorant d'oxydation et/ou un coupleur choisi parmi les paraphénylènediamines, les bases doubles, les para-aminophénols, les métaphénylènediamines, les méta-aminophénols, les métadiphénols, les bases et les coupleurs hétérocycliques, et les sels d'addition de ces composés avec un acide, et au moins un polymère amphotère comportant au moins une chaîne grasse ayant de 8 à 30 atomes de carbone.

Un autre objet de la présente invention porte sur une composition prête à l'emploi pour la teinture des fibres kératiniques, qui contient au moins un précurseur de colorant d'oxydation et/ou coupleur tel que défini ci-dessus et au moins un polymère amphotère comportant au moins une chaîne grasse, et en outre un agent oxydant.

Au sens de la présente invention, on entend par composition prête à l'emploi, toute composition destinée à être appliquée immédiatement sur les fibres kératiniques, et par chaîne grasse, on désigne une chaîne ayant de 8 à 30 atomes de carbone.

L'invention vise également un procédé de teinture des fibres kératiniques, et en particulier des fibres kératiniques humaines telles que les cheveux, consistant à appliquer sur les fibres au moins une composition colorante contenant, dans un milieu approprié pour la teinture, au moins un précurseur de colorant d'oxydation et/ou coupleur tel que défini ci-dessus, la couleur étant révélée à pH alcalin, neutre ou acide, à l'aide d'une composition oxydante contenant au moins un agent oxydant qui est mélangée juste au moment de l'emploi à la composition colorante ou qui est appliquée séquentiellement sans rinçage intermédiaire, au moins un polymère amphotère comportant au moins une chaîne grasse étant présent dans la composition colorante ou dans la composition oxydante ou dans chacune desdites compositions.

L'invention a également pour objet des dispositifs de teinture ou « kits » à plusieurs compartiments.

Un dispositif à 2 compartiments selon l'invention comprend un compartiment renfermant une composition colorante contenant, dans un milieu approprié pour la teinture, au moins un précurseur de colorant d'oxydation et/ou coupleur tel que défini ci-dessus, et un autre compartiment renfermant une composition oxydante contenant, dans un milieu approprié pour la teinture, un agent oxydant, le polymère amphotère comportant au moins une chaîne grasse étant présent dans la composition colorante ou la composition oxydante, ou dans chacune de ces compositions.

Mais d'autres caractéristiques, aspects, objets et avantages de l'invention apparaîtront encore plus clairement à la lecture de la description et des exemples qui suivent.

### Polymères amphotères comportant au moins une chaîne grasse avant de 8 à 30 atomes de carbone.

On désigne généralement par "polymères amphotères", des polymères qui comportent des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères utilisés selon l'invention comportent en outre au moins une chaîne grasse ayant de 8 à 30 atomes de carbone, et peuvent être choisis par exemple parmi les polymères dérivés d'acide polyaspartique comportant au moins une chaîne grasse ayant de 8 à 30 atomes de carbone, tels que ceux :
- décrits et préparés dans la demande de brevet EP- 0767 191 dont le contenu fait partie intégrante de la présente invention. De tels polymères sont préparés de façon connue, par réaction en milieu solvant, de polysuccinimide (PSI) sur des amines à chaîne grasse (en C₈-C₂₄), en la présence ou en l'absence de catalyseur basique tel que par exemple d'amines tertiaires aliphatiques, puis amphotérisation du produit obtenu par réaction avec un acide organique halogéné.
   Parmi les amines à chaîne grasse en C₈-C₂₄ que l'on fait réagir avec la PSI, on peut citer notamment l'octylamine, la nonylamine, la décylamine, la dodécylamine, la tétradécylamine, l'hexadécylamine, l'octadécylamine, l'octadécénylamine, l'eicosyldécylamine, l'octynylamine, la décénylamine, la dodécénylamine, la tétradécénylamine, l'hexadécénylamine, l'octadécénylamine, et l'eicosénylamine.
   Des exemples de tels polymères sont préparés par réaction de la PSI sur de la n-laurylamine ou de la n-stéarylamine en présence de N,N-diméthyl-1,3-propane diamine comme catalyseur basique, suivie d'une amphotérisation du produit obtenu par réaction avec le monochloroacétate de potassium. Ces polymères sont préparés avec de plus amples détails en pages 13 à 20 (lignes 1-4) et dans les exemples 1 à 5 en pages 28 à 34 (lignes 1-4) de la dite demande de brevet EP- 0767 191.
- décrits et préparés dans la demande de brevet EP- 0 884 344 dont le contenu fait partie intégrante de la présente invention. De tels polymères sont préparés par réaction en milieu solvant, d'ammoniac gazeux sur un monomaléate d'alkyl ou alcényl en C₈-C₂₄, sous pression réduite et à la température de 120-140°C pendant 4 à 6 heures.
   Les radicaux alkyl ou alcényl en C₈-C₂₄ peuvent notamment être choisis parmi les radicaux suivants linéaires ou ramifiés : décyl, dodécyl, tétradécyl, hexadécyl, octadécyl, oléyl.
   Des exemples de tels polymères comprennent les polymères à unités acide aspartique et à unités aspartate de décyl, les polymères à unités acide aspartique et à unités aspartate de dodécyl, les polymères à unités acide aspartique et à unités aspartate de cétyl, les polymères à unités acide aspartique et à unités aspartate de stéaryl, les polymères à unités acide aspartique et à unités n-décylaspartamide, décrits dans les exemples 1 à 6 de ladite demande de brevet.
- décrits et préparés dans la demande de brevet EP- 0 959 094 dont le contenu fait partie intégrante de la présente invention. De tels polymères sont préparés par réaction en milieu solvant, d'ammoniac gazeux sur un monoamide d'acide maléique, polyoxyalkyléné et modifié hydrophobe par une chaîne alkyle ou alcényle en C₈-C₃₀ linéaire ou ramifiée, éventuellement en mélange avec un monoester d'acide maléique.
   Un exemple de polymère ainsi préparé est décrit dans l'exemple 2 page 11 de ladite demande de brevet.
- décrits et préparés dans la demande de brevet EP- 0 959 090 dont le contenu fait partie intégrante de la présente invention. De tels polymères modifiés hydrophobes et de poids moléculaire élevé sont obtenus à partir de dérivés d'acide maléique et d'ammoniac gazeux et d'alcools ou d'amines di- ou poly-fonctionnels.
   Des exemples de copolymères à unités acide aspartique et aspartate de cétyle ou à unités acide aspartique et aspartate de cétyle sont respectivement donnés dans les exemples 3 et 5 de ladite demande de brevet.
- ou encore ceux décrits et préparés dans la demande de brevet EP- 0 959 091 dont le contenu fait partie intégrante de la présente invention. De tels polymères modifiés hydrophobes sont préparés à partir de monoester ou monoamide d'acide maléique et d'ammoniac gazeux.
   Des exemples de tels copolymères sont donnés dans les exemples 1, 2, 3, et 5 de ladite demande de brevet.

De préférence selon la présente invention, les polymères amphotères comportant au moins une chaîne grasse ayant de 8 à 30 atomes de carbone sont choisis parmi ceux comportant au moins un motif cationique non cyclique. Et plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.

Lesdits polymères amphotères à chaîne grasse préférés selon l'invention comprennent, ou sont préparés en copolymérisant :
1) au moins un monomère de formule (Ia) ou (Ib): 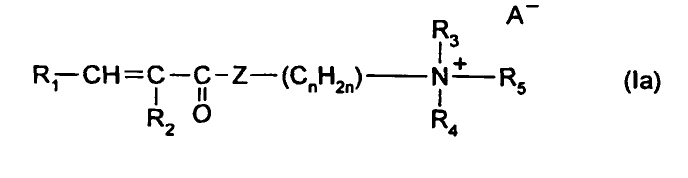 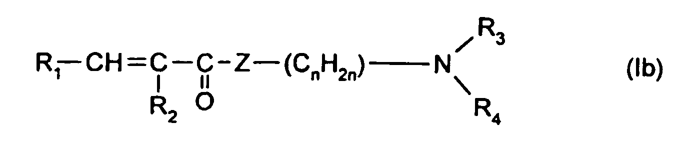 dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A⁻ est un anion issu d'un acide organique ou minéral, tel qu'un anion méthosulfate ou un halogénure tel que chlorure ou bromure;
2) au moins un monomère de formule (II)

   R₆-CH=CR₇-COOH

   dans laquelle, R₆ et R₇, identiques ou différents, représentent un atome d'hydrogène ou un radical méthyle;
   et
3) au moins un monomère de formule (III) :

   R₆-CH=CR₇-COXR₈
dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
l'un au moins des monomères de formule (Ia), (Ib) ou (III) comportant au moins une chaîne grasse.

Les monomères de formule (Ia) et (Ib) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,

ces monomères étant éventuellement quaternisés, par exemple par un halogénure d'alkyle en C₁-C₄ ou un sulfate de dialkyle en C₁-C₄.

Plus particulièrement, le monomère de formule (Ia) est choisi parmi le chlorure d'acrylamidopropyl triméthyl ammonium et le chlorure de méthacrylamidopropyl triméthyl ammonium.

Les monomères de formule (II) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (II) est l'acide acrylique.

Les monomères de formule (III) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères amphotères à chaîne grasse selon l'invention comprennent de préférence de 1 à 10 moles% du monomère comportant une chaîne grasse (monomère de formule (Ia), (Ib) ou (III)), et de préférence de 1,5 à 6 moles % .

Les poids moléculaires moyens en poids des polymères amphotères à chaîne grasse selon l'invention peuvent varier de 500 à 50.000.000 et sont de préférence compris entre 10.000 et 5 000 000.

Les polymères amphotères à chaîne grasse selon l'invention peuvent également contenir d'autre monomères tels que des monomères non ioniques et en particulier tels que les acrylates ou méthacrylates d'alkyle en C₁-C₄.

Des polymères amphotères à chaîne grasse selon l'invention sont par exemple décrits et préparés dans la demande de brevet WO9844012.

Parmi les polymères amphotères à chaîne grasse selon l'invention, on préfère les terpolymères acide acrylique/chlorure de (méth)acrylamidopropyl triméthyl ammonium/méthacrylate de stéaryle.

Le ou les polymère(s) amphotère(s) à chaîne grasse selon l'invention sont utilisés de préférence en une quantité comprise entre environ 0,05 et 10% en poids par rapport au poids total de la composition. Plus préférentiellement, cette quantité est comprise entre environ 0,1 et 5% en poids par rapport au poids total de la composition.

### Précurseurs de colorant d'oxydation

- **(I)** Les paraphénylènesdiamines sont choisies en particulier parmi celles de formule (I) suivante et leurs sels d'addition avec un acide : 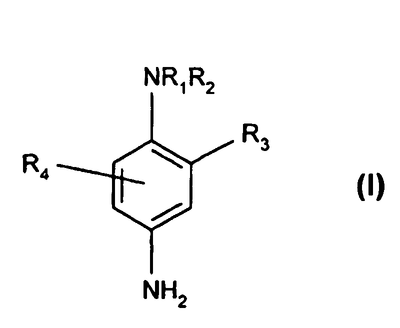 dans laquelle :
   R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   R₄ représente un atome d'hydrogène, d'halogène ou un radical alkyle en C₁-C₄ ;
   R₁ et R₂ peuvent également former avec l'atome d'azote qui les porte un hétérocycle azoté à 5 ou 6 chaînons éventuellement substitué par un ou plusieurs groupements alkyle, hydroxy ou uréido.

   Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β,hydroxyéthyl)-paraphénylènediamine, la N-(β,γ-dihydroxypropyl)-paraphénylènediamine, la N-(4'-aminophényl)-paraphènylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.
   Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- **(II)** Selon l'invention, on entend par bases doubles, les composés comportant au moins deux noyaux aromatiques sur lesquels sont portés des groupements amino et/ou hydroxyle.
   Parmi les bases doubles utilisables à titre de précurseurs de colorant d'oxydation dans les compositions tinctoriales conformes à l'invention, on peut notamment citer les composés répondant à la formule (II) suivante, et leurs sels d'addition avec un acide : 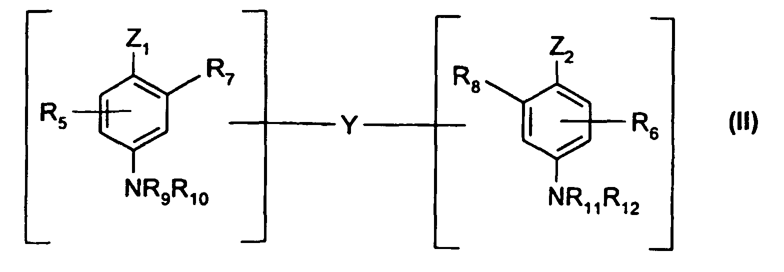 dans laquelle :
   - Z₁ et Z₂, identiques ou différents, représentent un radical hydroxyle ou -NH₂ pouvant être substitué par un radical alkyle en C₁-C₄ ou par un bras de liaison Y ;
   - le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
   - R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   - R₇, R₈, R₉, R₁₀, R₁₁ et R₁₂, identiques ou différents, représentent un atome d'hydrogène, un bras de liaison Y ou un radical alkyle en C₁-C₄ ;
   étant entendu que les composés de formule (II) ne comportent qu'un seul bras de liaison Y par molécule.
   Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.
   Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.
- **(III)** les para-aminophénols sont choisis en particulier parmi ceux répondant à la formule (III) suivante, et leurs sels d'addition avec un acide : 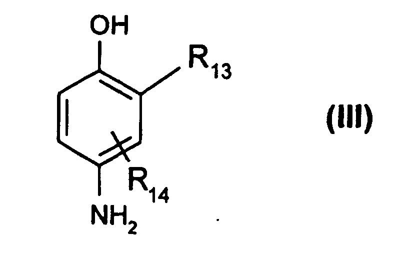 dans laquelle :
   R₁₃ représente un atome d'hydrogène, un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),

   Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- **(IV)** parmi les bases hétérocycliques utilisables à titre de précurseurs de colorant d'oxydation dans les compositions tinctoriales conformes à l'invention, on peut plus particulièrement citer les dérivés pyridiniques, les dérivés pyrimidiniques et pyrazolo-pyrimidiniques, les dérivés pyrazoliques, et leurs sels d'addition avec un acide.
   Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.
   Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol ; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino pyrazolo-[1,5-a]-pyrimidine; et leurs sels d'addition et leurs formes tautomères, lorsqu'il existe un équilibre tautomérique et leurs sels d'addition avec un acide.
   Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)- pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.
   Selon la présente invention, les précurseurs de colorant d'oxydation représentent de préférence de 0,0005 à 12% en poids environ du poids total de la composition et encore plus préférentiellement de 0,005 à 8% en poids environ de ce poids.

### Coupleurs

Les coupleurs utilisables dans la composition de teinture selon l'invention sont choisis parmi les méta-phénylènediamines, les méta-aminophénols, les métadiphénols, et les coupleurs hétérocycliques.

Parmi les coupleurs hétérocycliques, on peut citer les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Généralement le ou les coupleurs représentent de préférence de 0,0001 à 15% en poids environ du poids total de la composition tinctoriale prête à l'emploi et encore plus préférentiellement de 0,001 à 10% environ.

Les sels d'addition avec un acide de ces précurseurs de colorant d'oxydation et/ou coupleur sont notamment choisis parmi les chlorhydrates, les bromhydrates, les sulfates et les tartrates, les lactates et les acétates.

Selon une forme de réalisation préférée, la composition tinctoriale prête à l'emploi conforme à l'invention peut en outre renfermer un ou plusieurs colorants directs notamment pour modifier les nuances en les enrichissant de reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

Le milieu de la composition approprié pour la teinture, est de préférence un milieu aqueux constitué par de l'eau pouvant contenir avantageusement des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

La composition tinctoriale et/ou la composition oxydante peuvent en outre plus particulièrement contenir, au moins un agent tensio-actif dans la proportion d'au moins 0,01% en poids et de préférence un agent tensio-actif de nature non-ionique. Ces agents tensio-actifs sont choisis parmi :

### Les tensioactifs non ioniques :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### Les tensioactifs anioniques :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### Les tensioactifs amphotères ou zwittérioniques:

Les agents tensioactifs amphotères ou zwitterioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.
Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂-CONHCH₂CH₂-N(R₃)(R₄)(CH₂COO⁻)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

### Les tensioactifs cationiques :

Parmi les tensioactifs cationiques on peut citer en particulier (liste non limitative) : les sels d'amines grasses primaires, secondaires ou tertiaires, éventuellement polyoxyalkylénées; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,5 à 30% du poids total de la composition.

De préférence, selon l'invention, la composition tinctoriale et/ou la composition oxydante peuvent en outre plus particulièrement contenir, au moins un polymère cationique ou amphotère (différents de ceux à chaîne grasse selon l'invention) dans la proportion d'au moins 0,01% en poids par rapport au poids total de la composition.

### Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer :
**(1)** Les homopolymères ou copolymères dérivés d'esters ou d'amides acryliques ou méthacryliques et comportant au moins un des motifs de formules (I), (II), (III) ou (IV) suivantes: 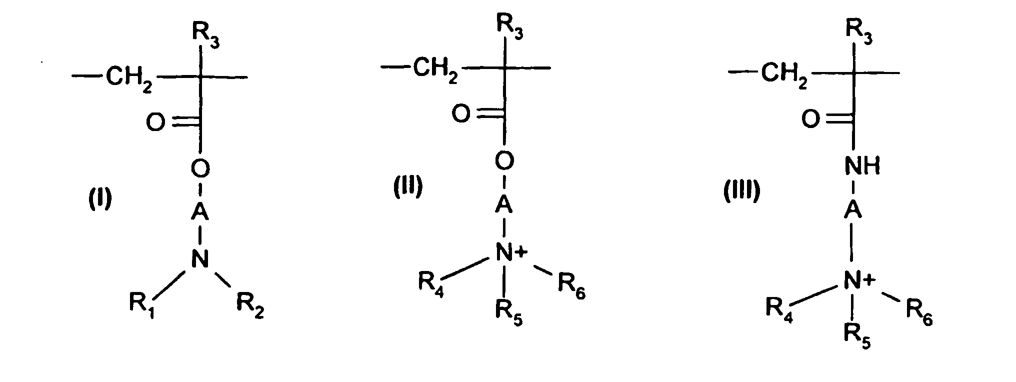 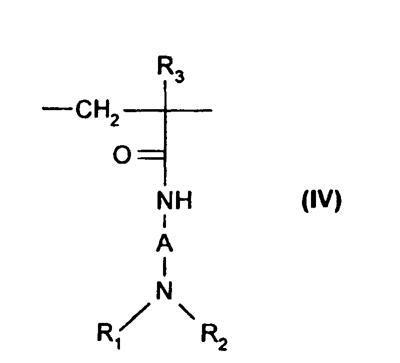 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate
   ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - et les copolymères vinylpyrrolidone / méthacrylamide de diméthylaminopropyle quaternisés tel que le produit vendu sous la dénomination "GAFQUAT HS 100" par la société ISP.
**(2)** Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
**(4)** Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quatemisation de ces polymères. De tels polymères sont notamment décrits dans les brevets français 2.162.025 et 2.280.361.
**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
**(7)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
**(9)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 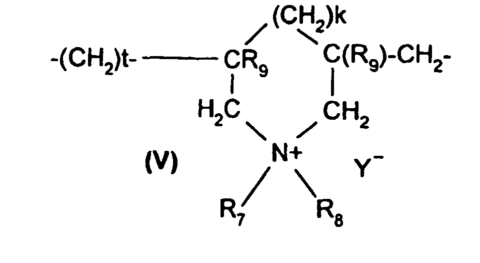 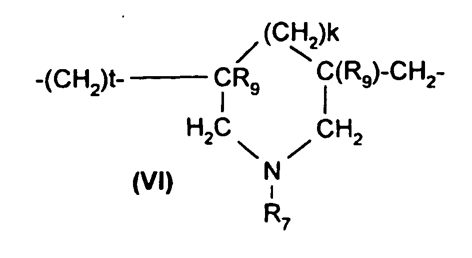 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
**(10)** Le polymère de diammonium quaternaire contenant des motifs récurrents répondant à la formule: 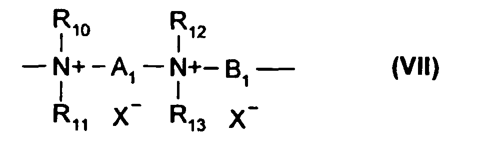 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH-R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un
   ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - (CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH-.

      De préférence, X⁻ est un anion tel que le chlorure ou le bromure.
      Ces polymères ont une masse moléculaire moyenne en nombre généralement comprise entre 1000 et 100000.
      Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
      On peut utiliser plus particulièrement les polymères qui sont constitués de motifs récurrents répondant à la formule (VIII) suivante: 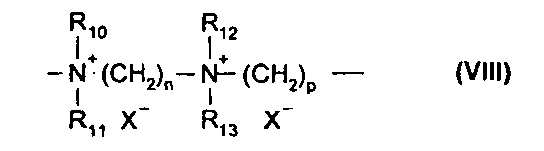 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
**(11)** Les polymères de polyammonium quaternaire constitués de motifs récurrents de formule (IX) : 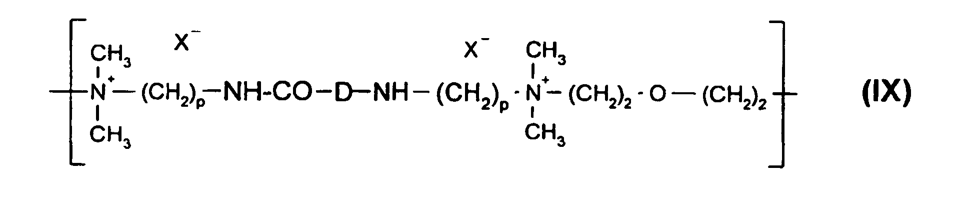 dans laquelle p désigne un nombre entier variant de 1 à 6 environ, D peut être nul ou peut représenter un groupement -(CH₂)ᵣ-CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
**(12)** Les polymères quaternaires de vinylpyrrolidone et de vinylimidazole tels que par exemple les produits commercialisés sous les dénominations Luviquat FC 905, FC 550 et FC 370 par la société B.A.S.F.
**(13)** Les polyamines comme le Polyquart H vendu par HENKEL, référencé sous le nom de " POLYETHYLENEGLYCOL (15) TALLOW POLYAMINE " dans le dictionnaire CTFA.
**(14)** Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de "SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de "SALCARE® SC 95" et " SALCARE® SC 96 " par la Société ALLIED COLLOIDS.

D'autres polymères cationiques utilisables dans le cadre de l'invention sont des polyalkyléneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 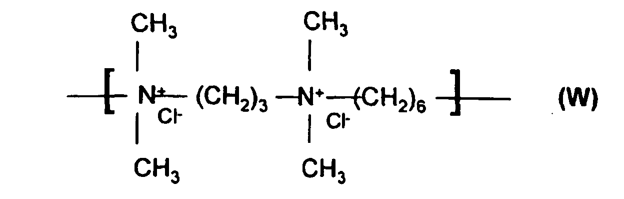 et notamment ceux dont le poids moléculaire, déterminé par chromatographie par perméation de gel, est compris entre 9500 et 9900 ; 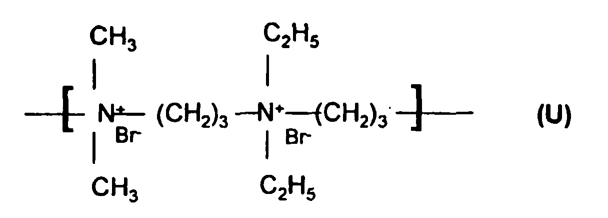 et notamment ceux dont le poids moléculaire, déterminé par chromatographie par perméation de gel, est d'environ 1200.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

### Polymères amphotères autres que ceux de l'invention

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appelations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylarninoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale : 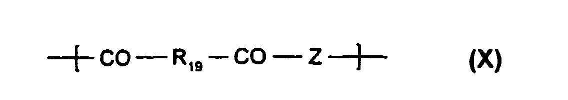 dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 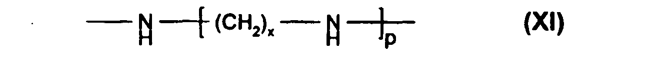 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 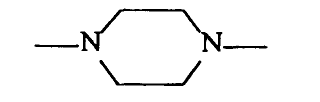
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 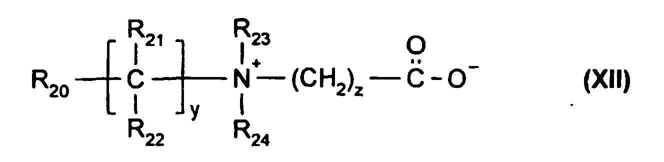 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.
(5) les polymères dérivés du chitosane comportant des motifs monomères répondant aux formules (XIII), (XIV), (XV) suivantes : 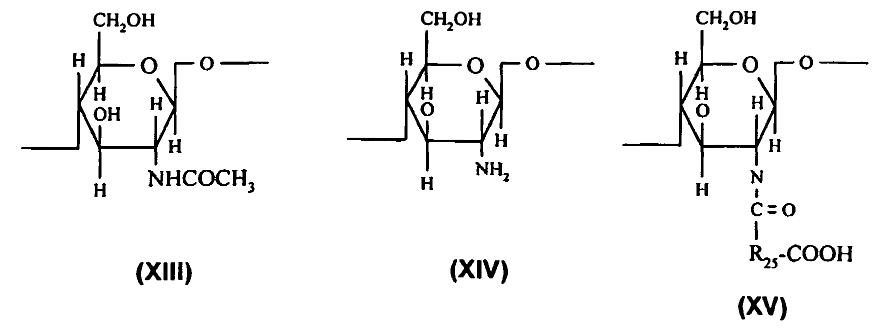 le motif (XIII) étant présent dans des proportions comprises entre 0 et 30%, le motif (XIV) dans des proportions comprises entre 5 et 50% et le motif F dans des proportions comprises entre 30 et 90%, étant entendu que dans ce motif (XV), R₂₅ représente un radical de formule : 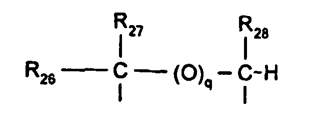 dans laquelle
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XI) tels que ceux décrits par exemple dans le brevet français 1 400 366 : 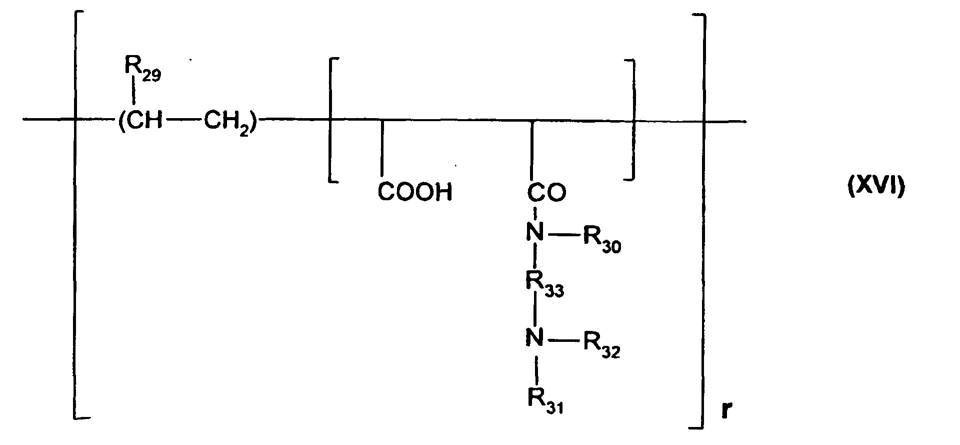 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂-, -CH₂-CH₂-CH₂-, -CH₂-CH(CH₃)-, R₃₁ ayant les significations mentionnées ci-dessus,
   ainsi que les homologues supérieurs de ces radicaux et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- **(XVII)**

      où D désigne un radical 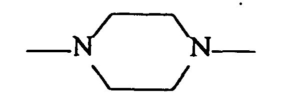 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- **(XVIII)**
   où D désigne un radical 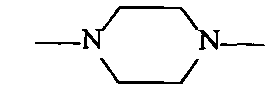 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié, partiellement par semiamidification avec une N,N-dialkylaminoalkylamine telle que la N,N-diméthylaminopropylamine ou par semiestérification avec une N,N-dialcanolamine.

Ces copolymères peuvent également comporter d'autres comonomères vinyliques tels que le vinylcaprolactame.

Les polymères amphotères particulièrement préférés sont ceux de la famille (1).

Selon l'invention, le ou les polymères amphotères autres que ceux à chaîne grasse selon l'invention peuvent représenter de 0,01 % à 10 % en poids, de préférence de 0,05 % à 5 % en poids, et encore plus préférentiellement de 0,1 % à 3 % en poids, du poids total de la composition.

La composition colorante peut encore contenir une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, etc....

Ladite composition peut également contenir des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 3% en poids par rapport au poids total de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition prête à l'emploi ou dans la composition oxydante, l'agent oxydant est choisi de préférence parmi le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition prête à l'emploi et appliquée sur les fibres kératiniques [composition résultant du mélange de la composition tinctoriale et de la composition oxydante], est généralement compris entre les valeurs 4 et 11. Il est de préférence compris entre 6 et 10, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 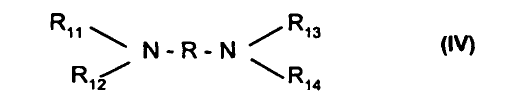 dans laquelle R est un reste propylène éventuellement substitué par un groupement hydroxyle ou un radical alkyle en C₁-C₄ ; R₁₁, R₁₂, R₁₃ et R₁₄, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer un mélange, réalisé extemporanément au moment de l'emploi, (composition prête à l'emploi) à partir des compositions tinctoriale et oxydante décrites ci-avant, sur les fibres kératiniques sèches ou humides, et à le laisser agir pendant un temps de pause variant, de préférence, de 1 à 60 minutes environ, et plus préférentiellement de 5 à 45 minutes environ, à rincer les fibres, puis éventuellement à les laver au shampooing, puis à les rincer à nouveau, et à les sécher.

Selon ledit procédé, les compositions tinctoriale et/ou oxydante peuvent contenir en outre au moins un polymère cationique ou amphotère (différent de ceux à chaîne grasse) et au moins un agent tensio-actif de préférence non-ionique.

Un exemple concret illustrant l'invention va maintenant être donné, sans pour autant présenter un caractère limitatif.

### EXEMPLE :

On a préparé la composition de teinture, conforme à l'invention, suivante :

| Composition colorante | % en poids |
|---|---|
| Tensioactifs non ioniques (alcools gras oxyéthylénés) | 32.5 |
| Glycérine | 3 |
| Acide oléique | 2 |
| Monoéthanolamine | 0.47 |
| Alcool oléique | 1.8 |
| Amide gras | 4 |
| Polymère amphotère à chaîne grasse selon l'invention [MAPTAC (49) / AA (49) / SMA (1,9994) / MBA (0,0006)] | 0.3 M.A* |
| Polymère cationique | 2.4 |
| Polymère amphotère différent de ceux de l'invention | 1.2 |
| Huiles végétales | 0.6 |
| Agent séquestrant, Anti-oxydant, Réducteur, Parfum | qs |
| Ammoniaque (20% NH₃) | 8 |
| 2-méthyl-5-hydroxyethyl aminophénol | 0.86 |
| p-aminophénol | 0.41 |
| 4-amino-2-hydroxytoluène | 0.57 |
| 6-hydroxyindole | 0.068 |
| p-phénylènediamine | 0.49 |
| Résorcinol | 0.1 |
| Eau | qsp 100 |

| | |
|---|---|
| M.A* désigne Matière Active MAPTAC= Chlorure de méthacrylamidopropyltriméthylammonium AA = Acide acrylique SMA = Méthacrylate de stéaryle MBA = N,N' méthylènebisacrylamide | |

Cette composition colorante après mélange avec du peroxyde d'hydrogène et application sur les cheveux permet d'obtenir une nuance soutenue, chromatique, faiblement sélective et tenace.

## Revendications

1. Composition pour la teinture d'oxydation des fibres kératiniques, en particulier des fibres kératiniques humaines telles que les cheveux, comprenant dans un milieu approprié pour la teinture, au moins un précurseur de colorant d'oxydation et/ou un coupleur choisi parmi les paraphénylènediamines, les bases doubles, les para-aminophénols, les métaphénylènediamines, les méta-aminophénols, les métadiphénols, les bases et les coupleurs hétérocycliques, et les sels d'addition de ces composés avec un acide, et au moins un polymère amphotère comportant au moins une chaîne grasse ayant de 8 à 30 atomes de carbone.

2. Composition selon la revendication 1, **caractérisée par le fait que** le polymère amphotère comportant au moins une chaîne grasse comporte également au moins un motif cationique non cyclique.

3. Composition selon les revendications 1 ou 2, **caractérisée par le fait que** ledit polymère amphotère comprend 1 à 20 moles% de monomère comportant une chaîne grasse, par rapport au nombre total de moles de monomères.

4. Composition selon l'une quelconque des revendications 1 à 3, **caractérisée par le fait que** les polymères amphotères comprennent :
1) au moins un monomère de formule (Ia) ou (Ib) suivante : 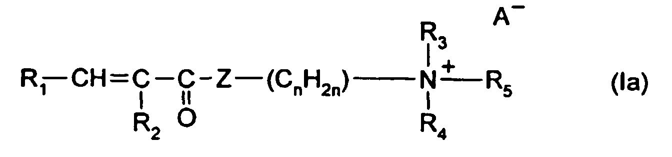 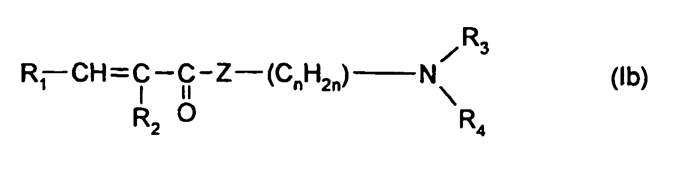 dans lesquelles R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
Z représente un groupe NH ou un atome d'oxygène,
n est un nombre entier de 2 à 5,
A⁻ est un anion issu d'un acide organique ou minéral;
2) au moins un monomère de formule (II)
R₆-CH=CR₇-COOH (II)
dans laquelle, R₆ et R₇, identiques ou différents, représentent un atome d'hydrogène ou un radical méthyle;
et
3) au moins un monomère de formule (III) :
R₆-CH=CR₇-COXR₈ (III)
dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone;
l'un au moins des monomères de formule (Ia), (Ib) ou (III) comportant au moins une chaîne grasse ayant de 8 à 30 atomes de carbone.

5. Composition selon la revendication 4, **caractérisée par le fait que** les monomères de formule (Ia) et (Ib) sont choisis dans le groupe constitué par le diméthylaminoéthylméthacrylate, le diméthylaminoéthylacrylate, le diéthylaminoéthylméthacrylate, le diéthylaminoéthylacrylate, le diméthylaminopropylméthacrylate, le diméthylaminopropylacrylate, le diméthylaminopropylméthacrylamide, le diméthylaminopropylacrylamide, éventuellement quatemisés.

6. Composition selon l'une quelconque des revendications 4 ou 5, **caractérisée par le fait que** le monomère de formule (Ia) est le chlorure d'acrylamidopropyl triméthyl ammonium ou le chlorure de méthacrylamidopropyl triméthyl ammonium.

7. Composition selon la revendication 4, **caractérisée par le fait que** le monomère de formule (II) est choisi dans le groupe constitué par l'acide acrylique, l'acide méthacrylique, l'acide crotonique et l'acide méthyl-2 crotonique.

8. Composition selon la revendication 4, **caractérisée par le fait que** le monomère de formule (III) est choisi dans le groupe constitué par des acrylates ou méthacrylates d'alkyle en C₁₂-C₂₂ et de préférence en C₁₆-C₁₈.

9. Composition selon l'une quelconque des revendications 1 à 8, **caractérisée par le fait que** lesdits polymères amphotères à chaîne grasse sont choisis parmi les terpolymères acide acrylique/chlorure de (méth)acrylamidopropyl triméthyl ammonium/méthacrylate de stéaryle.

10. Composition selon la revendication 1, **caractérisée par le fait que** les polymères amphotères à chaîne grasse sont choisis parmi les polymères dérivés d'acide polyaspartique.

11. Composition selon l'une quelconque des revendications précédentes, **caractérisée par le fait que** le polymère amphotère à chaîne grasse est utilisé en une quantité comprise entre 0,05 et 10% en poids par rapport au poids total de la composition.

12. Composition selon la revendication 11, **caractérisée par le fait que** le polymère amphotère à chaîne grasse est utilisé en une quantité comprise entre 0,1 et 5% en poids par rapport au poids total de la composition.

13. Composition selon la revendication 1, **caractérisée par le fait que** les paraphénylènediamines sont choisies parmi celles de formule (I) suivante et leurs sels d'addition avec un acide : 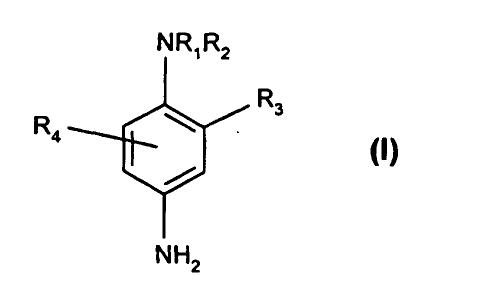 dans laquelle :
R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₃ représente un atome d'hydrogène, un atome d'halogène tel qu'un atome de chlore, un radical alkyle en C₁-C₄ sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un radical alkyle en C₁-C₄ ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido.

14. Composition selon la revendication 13, **caractérisée par le fait que** les radicaux azotés sont choisis parmi les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

15. Composition selon l'une quelconque des revendications 13 ou 14, **caractérisée par le fait que** les paraphénylènediamines sont choisies parmi : la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N, N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.

16. Composition selon la revendication 1, **caractérisée par le fait que** les bases doubles sont choisies parmi celles de formule (II) suivante, et leurs sels d'addition avec un acide : 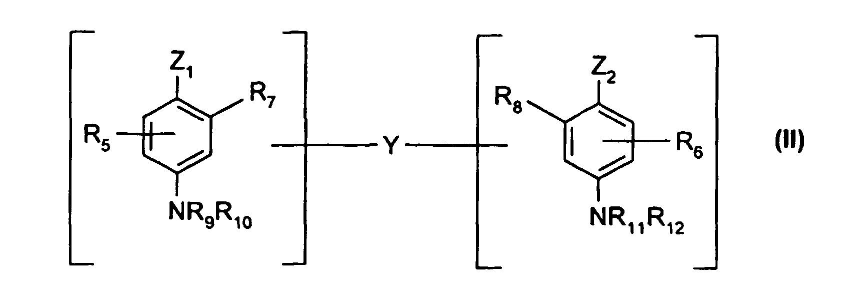 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

17. Composition selon la revendication 16, **caractérisée par le fait que** dans la formule (II) les groupements azotés sont choisis parmi les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

18. Composition selon l'une quelconque des revendications 16 ou 17, **caractérisée par le fait que** les bases doubles sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

19. Composition selon la revendication 1, **caractérisée par le fait que** les para-aminophénols sont choisis parmi ceux répondant à la formule (III) suivante, et leurs sels d'addition avec un acide : 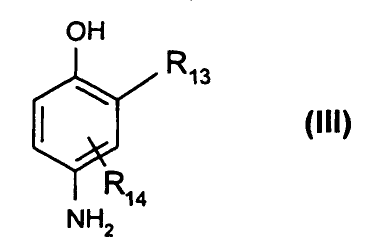 dans laquelle :
R₁₃ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

20. Composition selon la revendication 19, **caractérisée par le fait que** les para-aminophénols sont choisis parmi le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

21. Composition selon la revendication 1, **caractérisée par le fait que** les bases hétérocycliques sont choisies parmi les dérivés pyridiniques, les dérivés pyrimidiniques et pyrazolo-pyrimidiniques, les dérivés pyrazoliques, et leurs sels d'addition avec un acide.

22. Composition selon la revendication 21, **caractérisée par le fait que** les dérivés pyridiniques sont choisis parmi la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

23. Composition selon la revendication 21, **caractérisée par le fait que** les dérivés pyrimidiniques sont choisis parmi la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques sont choisis parmi la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino pyrazolo-[1,5-a]-pyrimidine; et leurs sels d'addition et leurs formes tautomères, lorsqu'il existe un équilibre tautomérique et leurs sels d'addition avec un acide.

24. Composition selon la revendication 21, **caractérisée par le fait que** les dérivés pyrazoliques sont choisis parmi le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)- pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3.5-diamino-1-méthyl-4-méthylamino-pyrazole, le 3,5-diamino-4-(β-hydroxyéthyl)amino-1-méthyl-pyrazole, et leurs sels d'addition avec un acide.

25. Composition selon l'une quelconque des revendications précédentes, **caractérisée par le fait que** les précurseurs de colorant d'oxydation sont présents dans des concentrations allant de 0,0005 à 12% en poids par rapport au poids total de la composition.

26. Composition selon la revendication 1, **caractérisée par le fait que** les coupleurs sont choisis parmi les méta-phénylènediamines, les méta-aminophénols, les métadiphénols, les coupleurs hétérocycliques, et leurs sels d'addition avec un acide.

27. Composition selon la revendication 26, **caractérisée par le fait que** les coupleurs sont choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

28. Composition selon l'une quelconque des revendications précédentes, **caractérisée par le fait que** les coupleurs sont présents dans des concentrations allant de 0,0001 à 10% en poids par rapport au poids total de la composition.

29. Composition selon l'une quelconque des revendications 1 et 13 à 28, **caractérisée par le fait que** les sels d'addition avec un acide des précurseurs de colorants d'oxydation et des coupleurs sont choisis parmi les chlorhydrates, les bromhydrates, les sulfates, les tartrates, les lactates et les acétates.

30. Composition selon l'une quelconque des revendications précédentes, **caractérisée par le fait qu'**elle contient en outre des colorants directs dans la proportion de 0,001 à 20% en poids par rapport au poids total de la composition.

31. Composition selon l'une quelconque des revendications précédentes, **caractérisée par le fait qu'**elle contient en outre au moins un polymère cationique ou un polymère amphotère différent des polymères amphotères à chaîne grasse définis à l'une quelconque des revendications 1 à 10.

32. Composition selon la revendication 31, **caractérisée par le fait que** le polymère cationique est un polyammonium quaternaire constitué de motifs récurrents répondant à la formule (W) suivante :

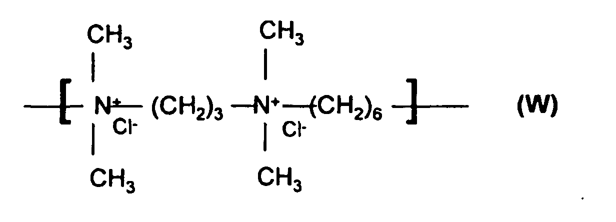

33. Composition selon la revendication 31, **caractérisée par le fait que** le polymère cationique est un polyammonium quaternaire constitué de motifs récurrents répondant à la formule (U) suivante :

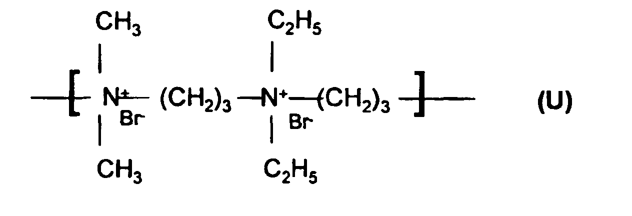

34. Composition selon la revendication 31, **caractérisée par le fait que** le polymère amphotère est un copolymère comprenant au moins comme monomères de l'acide acrylique et un chlorure de diméthyldiallylammonium.

35. Composition selon l'une quelconque des revendications 31 à 34, **caractérisée par le fait que** le ou les polymères cationiques ou les polymères amphotères différents de ceux à chaîne grasse selon l'invention représentent de 0,01 % à 10 % du poids total de la composition.

36. Composition selon l'une quelconque des revendications précédentes, **caractérisée par le fait qu'**elle contient au moins un tensioactif choisi parmi les tensioactifs anioniques, cationiques, non ioniques ou amphotères.

37. Composition selon la revendication 36, **caractérisée par le fait que** le ou les tensio-actifs sont non-ioniques.

38. Composition selon les revendications 36 ou 37, **caractérisée par le fait que** les tensioactifs représentent 0,01 à 40% du poids total de la composition.

39. Composition selon l'une quelconque des revendications précédentes, **caractérisée par le fait qu'**elle contient en outre au moins un agent réducteur, dans des quantités allant de 0,05 à 3% en poids par rapport au poids total de la composition.

40. Composition selon l'une quelconque des revendications précédentes, prête à l'emploi, **caractérisée par le fait qu'**elle contient en outre un agent oxydant.

41. Composition selon la revendication 40, **caractérisée par le fait qu'**elle possède un pH allant de 4 à 11.

42. Composition selon la revendication 40, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

43. Composition selon la revendication 42, **caractérisée par le fait que** l'agent oxydant est le peroxyde d'hydrogène.

44. Composition selon la revendication 43, **caractérisée par le fait que** l'agent oxydant est une solution d'eau oxygénée dont le titre varie de 1 à 40 volumes.

45. Procédé de teinture des fibres kératiniques et en particulier des fibres kératiniques humaines telles que les cheveux, **caractérisé par le fait qu'**il consiste à appliquer sur les fibres au moins une composition colorante contenant, dans un milieu approprié pour la teinture, au moins un précurseur de colorant d'oxydation et/ou un coupleur choisi parmi les paraphénylènediamines, les bases doubles, les para-aminophénols, les métaphénylènediamines, les méta-aminophénols, les métadiphénols, les bases et les coupleurs hétérocycliques, et les sels d'addition de ces composés avec un acide,
la couleur étant révélée à pH alcalin, neutre ou acide à l'aide d'une composition oxydante contenant au moins un agent oxydant, qui est mélangée juste au moment de l'emploi à la composition colorante ou qui est appliquée séquentiellement sans rinçage intermédiaire, au moins un polymère amphotère comportant au moins une chaîne grasse défini selon les revendications 1 à 10 étant présent dans la composition colorante ou dans la composition oxydante ou dans chacune desdites compositions.

46. Procédé selon la revendication 30, **caractérisé par le fait qu'**il consiste à appliquer sur les fibres kératiniques sèches ou humides, la composition prête à l'emploi, réalisée extemporanément au moment de l'emploi à partir des compositions colorante et oxydante, à la laisser agir pendant un temps de pause variant de 1 à 60 minutes environ, et de préférence de 5 à 45 minutes, à rincer les fibres, puis éventuellement à les laver au shampooing, puis à les rincer à nouveau, et à les sécher.

47. Dispositif à plusieurs compartiments ou « Kit » pour la teinture des fibres kératiniques et en particulier des fibres kératiniques humaines telles que les cheveux, **caractérisé par le fait qu'**il comporte au moins deux compartiments, dont l'un d'entre eux renferme une composition colorante contenant, dans un milieu approprié pour la teinture, au moins un précurseur de colorant d'oxydation et/ou un coupleur choisi parmi les paraphénylènediamines, les bases doubles, les para-aminophénols, les métaphénylènediamines, les méta-aminophénols, les métadiphénols, les bases et les coupleurs hétérocycliques, et les sels d'addition de ces composés avec un acide,
et un autre une composition oxydante comprenant, dans un milieu approprié pour la teinture, un agent oxydant, un polymère amphotère comportant au moins une chaîne grasse tel que défini à l'une quelconque des revendications 1 à 10 étant présent dans la composition colorante ou la composition oxydante ou dans chacune des deux compositions.

## Patentansprüche

1. Zusammensetzung zum oxidativen Färben von Keratinfasern, insbesondere menschlichen Keratinfasern, beispielsweise zum Färben der Haare, die in einem zum Färben geeigneten Medium mindestens ein Farbstoffvorprodukt eines Oxidationsfarbstoffes und/oder einen Kuppler, die unter den p-Phenylendiaminen, Doppelbasen, p-Aminophenolen, m-Phenylendiaminen, m-Aminophenolen, m-Dihydroxybenzolen, heterocyclischen Basen und heterocyclischen Kupplern und den Additionssalzen dieser Verbindungen mit einer Säure ausgewählt sind, und mindestens ein amphoteres Polymer enthält, das mindestens eine Fettkette mit 8 bis 30 Kohlenstoffatomen aufweist.

2. Zusammensetzung nach Anspruch 1, **dadurch gekennzeichnet, dass** das amphotere Polymer, das mindestens eine Fettkette aufweist, ferner mindestens eine nicht cyclische kationische Einheit enthält.

3. Zusammensetzung nach den Ansprüchen 1 oder 2, **dadurch gekennzeichnet, dass** das amphotere Polymer 1 bis 20 Mol-% Monomer mit Fettkette, bezogen auf die Molzahl der Monomere insgesamt, enthält.

4. Zusammensetzung nach einem der Ansprüche 1 bis 3, **dadurch gekennzeichnet, dass** die amphoteren Polymere umfassen:
1) mindestens ein Monomer der folgenden Formel (Ia) oder (Ib): 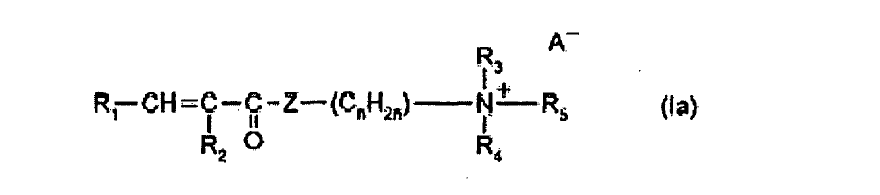 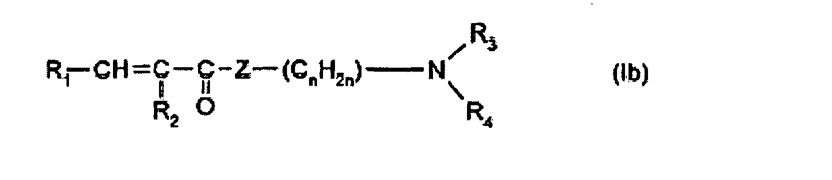 worin die Gruppen R₁ und R₂, die gleich oder verschieden sind, ein Wasserstoffatom oder Methyl bedeuten, die Gruppen R₃, R₄ und R₅, die gleich oder verschieden sind, eine geradkettige oder verzweigte Alkylgruppe mit 1 bis 30 Kohlenstoffatomen bedeuten,
Z die Gruppe NH oder ein Sauerstoffatom ist,
n eine ganze Zahl von 2 bis 5 bedeutet, und
A- ein Anion ist, das von einer organischen oder anorganischen Säure stammt;
2) mindestens ein Monomer der Formel (II)
R₆-CH=CR₇-COOH (II)
worin die Gruppen R₆ und R₇, die gleich oder verschieden sind, ein Wasserstoffatom oder Methyl bedeuten; und
3) mindestens ein Monomer der Formel (III):
R₆-CH=CR₇-COXR₈ (III)
worin die Gruppen R₆ und R₇, die gleich oder verschieden sind, ein Wasserstoffatom oder Methyl bedeuten; X ein Sauerstoffatom oder Stickstoffatom ist und R₈ eine geradkettige oder verzweigte Alkylgruppe mit 1 bis 30 Kohlenstoffatomen bedeutet;
wobei mindestens ein Monomer der Formel (Ia), (Ib) oder (III) mindestens eine Fettkette mit 8 bis 30 Kohlenstoffatomen aufweist.

5. Zusammensetzung nach Anspruch 4, **dadurch gekennzeichnet, dass** die Monomere der Formel (Ia) und (Ib) unter Dimethylaminoethylmethacrylat, Dimethylaminoethylacrylat, Diethylaminoethylmethacrylat, Diethylaminoethylacrylat, Dimethylaminopropylmethacrylat, Dimethylaminopropylacrylat, Dimethylaminopropylmethacrylamid und Dimethylaminopropylacrylamid ausgewählt sind, die gegebenenfalls quaternisiert sind.

6. Zusammensetzung nach einem der Ansprüche 4 oder 5, **dadurch gekennzeichnet, dass** das Monomer der Formel (Ia) das Acrylamidopropyltrimethylammoniumchlorid oder Methacrylamidopropyltrimethylammoniumchlorid ist.

7. Zusammensetzung nach Anspruch 4, **dadurch gekennzeichnet, dass** das Monomer der Formel (II) unter Acrylsäure, Methacrylsäure, Crotonsäure oder 2-Methylcrotonsäure ausgewählt ist.

8. Zusammensetzung nach Anspruch 4, **dadurch gekennzeichnet, dass** das Monomer der Formel (III) unter den C₁₂₋₂₂-Alkylacrylaten oder C₁₂₋₂₂-Alkylmethacrylaten ausgewählt ist, wobei die Alkylgruppe vorzugsweise 16 bis 18 Kohlenstoffatome aufweist.

9. Zusammensetzung nach einem der Ansprüche 1 bis 8, **dadurch gekennzeichnet, dass** die amphoteren Polymere mit Fettkette unter den Acrylsäure/(Meth)acrylamidopropyltrimethyl-ammoniumchlorid/Stearylmethacrylat-Terpolymeren ausgewählt sind.

10. Zusammensetzung nach Anspruch 1, **dadurch gekennzeichnet, dass** die amphoteren Polymere mit Fettkette unter den Polymeren ausgewählt sind, die von Polyasparaginsäure abgeleitet sind.

11. Zusammensetzung nach einem der vorhergehenden Ansprüche, **dadurch gekennzeichnet, dass** das amphotere Polymer mit Fettkette in einer Menge von 0,05 bis 10 Gew.-%, bezogen auf das Gesamtgewicht der Zusammensetzung, verwendet wird.

12. Zusammensetzung nach Anspruch 11, **dadurch gekennzeichnet, dass** das amphotere Polymer mit Fettkette in einer Menge von 0,1 bis 5 Gew.-%, bezogen auf das Gesamtgewicht der Zusammensetzung, verwendet wird.

13. Zusammensetzung nach Anspruch 1, **dadurch gekennzeichnet, dass** die p-Phenylendiamine unter den Verbindungen der folgenden Formel (I) und deren Additionssalzen mit einer Säure ausgewählt sind: 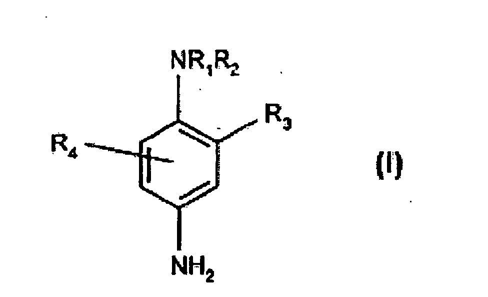 worin bedeuten:
R₁ ein Wasserstoffatom, C₁₋₄-Alkyl, C₁₋₄-Monohydroxyalkyl, C₂₋₄-Polyhydroxyalkyl, Alkoxy(C₁₋₄)alkyl(C₁₋₄), eine mit einer stickstoffhaltigen Gruppe substituierte C₁₋₄-Alkylgruppe, Phenyl oder 4'-Aminophenyl;
R₂ ein Wasserstoffatom, C₁₋₄-Alkyl, C₁₋₄-Monohydroxyalkyl, C₂₋₄-Polyhydroxyalkyl, Alkoxy(C₁₋₄)alkyl(C₁₋₄) oder eine mit einer stickstoffhaltigen Gruppe substituierte C₁₋₄-Alkylgruppe;
R₃ ein Wasserstoffatom, ein Halogenatom, wie Chlor, C₁₋₄-Alkyl, Sulfo, Carboxy, C₁₋₄-Monohydroxyalkyl, C₁₋₄-Hydroxyalkoxy, C₁₋₄-Acetylaminoalkoxy, C₁₋₄-Mesylaminoalkoxy oder C₁₋₄-Carbamoylaminoalkoxy,
R₄ ein Wasserstoffatom, ein Halogenatom oder C₁₋₄-Alkyl;
wobei die Gruppen R₁ und R₂ auch mit dem Stickstoffatom, an das sie gebunden sind, einen stickstoffhaltigen, 5- oder 6-gliedrigen Heterocyclus bilden können, der gegebenenfalls mit einer oder mehreren Gruppen Alkyl, Hydroxy oder Ureido substituiert ist.

14. Zusammensetzung nach Anspruch 13, **dadurch gekennzeichnet, dass** die stickstoffhaltigen Gruppen unter den Gruppen Amino, Monoalkyl(C₁₋₄)amino, Dialkyl(C₁₋₄)amino, Trialkyl(C₁₋₄)amino, Monohydroxyalkyl(C₁₋₄)amino, Imidazolinium und Ammonium ausgewählt sind.

15. Zusammensetzung nach einem der Ansprüche 13 oder 14, **dadurch gekennzeichnet, dass** die p-Phenylendiamine unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methylanilin, N,N-Bis(β-hydroxyethyl)-p-phenylendiamin, 4-N,N-Bis(β-hydroxyethylamino-2-methylanilin, 4-N,N-Bis(β-hydroxyethylamino-2-chloranilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl-β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylaminoethyloxy-p-phenylendiamin, N-(β-Methoxyethyl)-p-phenylendiamin, 2-Methyl-1-N-β-hydroxyethyl-p-phenylendiamin und deren Additionssalze mit einer Säure ausgewählt sind.

16. Zusammensetzung nach Anspruch 1, **dadurch gekennzeichnet, dass** die Doppelbasen unter den Verbindungen der folgenden Formel (II) und deren Additionssalzen mit einer Säure ausgewählt sind: 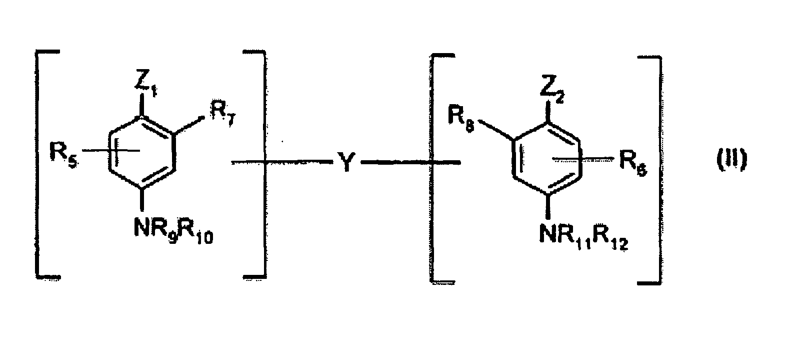 worin bedeuten:
- die Gruppen Z₁ und Z₂, die gleich oder verschieden sind, eine Hydroxygruppe oder eine NH₂-Gruppe, die mit einer C₁₋₄-Alkylgruppe oder einer Verbindungsgruppe Y substituiert sein kann;
- die Verbindungsgruppe Y eine geradkettige oder verzweigte Alkylengruppe mit 1 bis 14 Kohlenstoffatomen, die durch eine oder mehrere stickstoffhaltige Gruppen und/oder ein oder mehrere Heteroatome, wie Sauerstoff, Schwefel oder Stickstoff, unterbrochen oder abgeschlossen werden kann und gegebenenfalls mit einer oder mehreren Hydroxygruppen oder C₁₋₆-Alkoxygruppen substituiert ist;
- R₅ und R₆ ein Wasserstoffatom, ein Halogenatom, C₁₋₄-Alkyl, C₁₋₄-Monohydroxyalkyl, C₂₋₄-Polyhydroxyalkyl, C₁₋₄-Aminoalkyl oder eine Verbindungsgruppe Y;
- die Gruppen R₇, R₈, R₉, R₁₀, R₁₁ und R₁₂, die gleich oder verschieden sind, ein Wasserstoffatom, eine Verbindungsgruppe Y oder C₁₋₄-Alkyl;
mit der Maßgabe, dass die Verbindungen der Formel (II) nur eine Verbindungsgruppe Y pro Molekül aufweisen.

17. Zusammensetzung nach Anspruch 16, **dadurch gekennzeichnet, dass** in der Formel (II) die stickstoffhaltigen Gruppen unter Amino, Monoalkyl(C₁₋₄)amino, Dialkyl(C₁₋₄)amino, Trialkyl(C₁₋₄)amino, Monohydroxyalkyl(C₁₋₄)amino, Imidazolinium und Ammonium ausgewählt sind.

18. Zusammensetzung nach einem der Ansprüche 16 oder 17, **dadurch gekennzeichnet, dass** die Doppelbasen unter N,N'-Bis(β-hydroxyethyl)-N,N'-bis(4'-aminophenyl)-1,3-diaminopropanol, N,N'-Bis(β-hydroxyethyl)-N,N'-bis (4'-aminophenyl)-ethylendiamin, N,N'-Bis(4-aminophenyl)-tetramethylendiamin, N,N'-Bis-(β-hydroxyethyl)-N,N'-bis(4-aminophenyl)-tetramethylendiamin, N,N'-Bis(4-methylaminophenyl)-tetramethylendiamin, N,N'-Bis-(ethyl)-N,N'-bis(4'-amino, 3'-methylphenyl)-ethylendiamin, 1,8-Bis-(2,5-diaminophenoxy)-3,5-dioxaoctan und deren Additionssalzen mit einer Säure ausgewählt sind.

19. Zusammensetzung nach Anspruch 1, **dadurch gekennzeichnet, dass** die p-Aminophenole unter den Verbindungen der folgenden Formel (III) und deren Additionssalzen mit einer Säure ausgewählt sind: 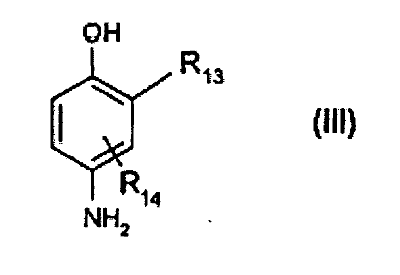 worin bedeuten:
R₁₃ ein Wasserstoffatom, ein Halogenatom, wie Fluor, C₁₋₄-Alkyl, C₁₋₄-Monohydroxyalkyl, Alkoxy(C₁₋₄)alkyl(C₁₋₄), C₁₋₄-Aminoalkyl oder Hydroxyalkyl(C₁₋₄)aminoalkyl(C₁₋₄),
R₁₄ ein Wasserstoffatom, ein Halogenatom, wie Fluor, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder Alkoxy(C₁₋₄)alkyl(C₁₋₄).

20. Zusammensetzung nach Anspruch 19, **dadurch gekennzeichnet, dass** die p-Aminophenole unter 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol und deren Additionssalzen mit einer Säure ausgewählt sind.

21. Zusammensetzung nach Anspruch 1, **dadurch gekennzeichnet, dass** die heterocyclischen Basen unter den Pyridinderivaten, Pyrimidinderivaten, Pyrazolo-pyrimidinderivaten, Pyrazolderivaten und deren Additionssalzen mit einer Säure ausgewählt sind.

22. Zusammensetzung nach Anspruch 21, **dadurch gekennzeichnet, dass** die Pyridinderivate unter 2,5-Diaminopyridin, 2-(4-Methoxyphenyl)amino-3-aminopyridin, 2,3-Diamino-6-methoxy-pyridin, 2-(β-Methoxyethyl)amino-3-amino-6-methoxypyridin, 3,4-Diaminopyridin und deren Additionssalzen mit einer Säure ausgewählt sind.

23. Zusammensetzung nach Anspruch 21, **dadurch gekennzeichnet, dass** die Pyrimidinderivate unter 2,4,5,6-Tetraaminopyrimidin, 4-Hydroxy-2,5,6-triaminopyrimidin, 2-Hydroxy-4,5,6-triamino-pyrimidin, 2,4-Dihydroxy-5,6-diaminopyrimidin und 2,5,6-Triaminopyrimidin und die Pyrazolo-pyrimidinderivate unter Pyrazolo[1,5-a]pyrimidin-3,7-diamin, 2,5-Dimethyl-pyrazolo[1,5-a]-pyrimidin-3,7-diamin, Pyrazolo[1,5-a]pyrimidin-3,5-diamin, 2,7-Dimethyl-pyrazolo[1,5-a]pyrimidin-3,5-diamin, 3-Amino-pyrazolo[1,5-a]pyrimidin-7-ol, 3-Aminopyrazolo-[1,5-a]-pyrimidin-5-ol, 2-(3-Amino-pyrazolo[1,5-a]pyrimidin-7-ylamino)-ethanol, 2-(7-Amino-pyrazolo-[1,5-a]pyrimidin-3-ylamino)-ethanol, 2-[(3-Amino-pyrazolo[1,5-a]pyrimidin-7-yl)-(2-hydroxyethyl)amino]-ethanol, 2-[(7-Amino-pyrazolo[1,5]-pyrimidin-3-yl)-(2-hydroxyethyl)amino]-ethanol, 5,6-Dimethyl-pyrazolo[1,5-a]-pyrimidin-3,7-diamin, 2,6-Dimethyl-pyrazolo[1,5-a]pyrimidin-3,7-diamin, 2,5,N7,N7-Tetramethyl-pyrazolo[1,5-a]pyrimidin-3,7-diamin, 3-Amino-5-methyl-7-imidazolylpropylamino-pyrazolo[1,5-a]pyrimidin und deren Additionssalzen und deren tautomeren Formen, falls ein tautomeres Gleichgewicht vorliegt, und deren Additionssalzen mit einer Säure ausgewählt sind.

24. Zusammensetzung nach Anspruch 21, **dadurch gekennzeichnet, dass** die Pyrazolderivate unter 4,5-Diamino-1-methylpyrazol, 3,4-Diaminopyrazol, 4,5-Diamino-1-(4'-chlorbenzyl)-pyrazol, 4,5-Diamino-1,3-dimethylpyrazol, 4, 5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-*t*-butyl-1-methylpyrazol, 4, 5-Diamino-1-*t*-butyl-3-methylpyrazol, 4,5-Diamino-1-(β-hydroxyethyl)-3-methylpyrazol, 4,5-Diamino-1-(β-hydroxyethyl)-pyrazol, 4,5-Diamino-1-ethyl-3-methylpyrazol, 4, 5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol, 3,5-Diamino-4-(β-hydroxyethyl)amino-1-methylpyrazol und deren Additionssalzen mit einer Säure ausgewählt sind.

25. Zusammensetzung nach einem der vorhergehenden Ansprüche, **dadurch gekennzeichnet, dass** die Farbstoffvorprodukte der Oxidationsfarbstoffe in Konzentrationen von 0,0005 bis 12 Gew.-%, bezogen auf das Gesamtgewicht der Zusammensetzung, enthalten sind.

26. Zusammensetzung nach Anspruch 1, **dadurch gekennzeichnet, dass** die Kuppler unter den m-Phenylendiaminen, m-Aminophenolen, m-Dihydroxybenzolen, heterocyclischen Kupplern und deren Additionssalzen mit einer Säure ausgewählt sind.

27. Zusammensetzung nach Anspruch 26, **dadurch gekennzeichnet, dass** die Kuppler unter 2,4-Diamino-1-(β-hydroxyethyloxy)-benzol, 2-Methyl-5-aminophenol, 5-N-(β-Hydroxyethyl)amino-2-methylphenol, 3-Aminophenol, 1,3-Dihydroxybenzol, 1,3-Dihydroxy-2-methylbenzol, 4-Chlor-1,3-dihydroxybenzol, 2-Amino-4-(β-hydroxyethylamino)-1-methoxybenzol, 1,3-Diaminobenzol, 1,3-Bis-(2,4-diaminophenoxy)-propan, Sesamol, 1-Amino-2-methoxy-4,5-methylendioxybenzol, 6-Hydroxyindol, 4-Hydroxyindol, 4-Hydroxy-N-methylindol, 6-Hydroxyindolin, 2,6-Dihydroxy-4-methylpyridin, 1-H-3-Methylpyrazol-5-on, 1-Phenyl-3-methylpyrazol-5-on, 2-Amino-3-hydroxypyridin, 3,6-Dimethyl-pyrazolo-[3,2-c]-1,2,4-triazol, 2,5-Dimethylpyrazolo-[1,5-b]-1,2,4-triazol und deren Additionssalzen mit einer Säure ausgewählt sind.

28. Zusammensetzung nach einem der vorhergehenden Ansprüche, **dadurch gekennzeichnet, dass** die Kuppler in Konzentrationen von 0,0001 bis 10 Gew.-%, bezogen auf das Gesamtgewicht der Zusammensetzung, enthalten sind.

29. Zusammensetzung nach einem der Ansprüche 1 und 13 bis 28, **dadurch gekennzeichnet, dass** die Additionssalze der Farbstoffvorprodukte von Oxidationsfarbstoffen und Kuppler mit einer Säure unter den Hydrochloriden, Hydrobromiden, Sulfaten, Tartraten, Lactaten und Acetaten ausgewählt sind.

30. Zusammensetzung nach einem der vorhergehenden Ansprüche, **dadurch gekennzeichnet, dass** sie ferner Direktfarbstoffe in Mengenanteilen von 0,001 bis 20 Gew.-%, bezogen auf das Gesamtgewicht der Zusammensetzung, enthält.

31. Zusammensetzung nach einem der vorhergehenden Ansprüche, **dadurch gekennzeichnet, dass** sie ferner mindestens ein kationisches Polymer oder ein amphoteres Polymer enthält, das von den amphoteren Polymeren mit Fettkette nach einem der Ansprüche 1 bis 10 verschieden ist.

32. Zusammensetzung nach Anspruch 31, **dadurch gekennzeichnet, dass** das kationische Polymer ein quartäres Polyammoniumpolymer ist, das aus wiederkehrenden Einheiten der folgenden Formel (W) besteht:

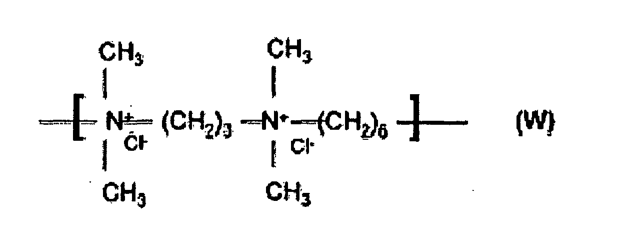

33. Zusammensetzung nach Anspruch 31, **dadurch gekennzeichnet, dass** das kationische Polymer ein quartäres Polyammoniumpolymer ist, das aus wiederkehrenden Einheiten der folgenden Formel (U) besteht:

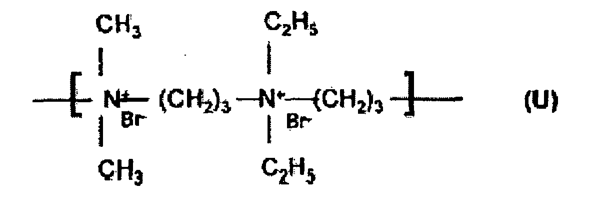

34. Zusammensetzung nach Anspruch 31, **dadurch gekennzeichnet, dass** das amphotere Polymer ein Copolymer ist, das als Monomere zumindest Acrylsäure und ein Dimethyldiallylammoniumchlorid enthält.

35. Zusammensetzung nach einem der Ansprüche 31 bis 34, **dadurch gekennzeichnet, dass** das oder die kationische(n) Polymer(e) oder amphoteren Polymere, die von den Polymeren mit Fettkette gemäß der Erfindung verschieden sind, 0,01 bis 10 % des Gesamtgewichts der Zusammensetzung ausmachen.

36. Zusammensetzung nach einem der vorhergehenden Ansprüche, **dadurch gekennzeichnet, dass** sie mindestens einen grenzflächenaktiven Stoff enthält, der unter den anionischen, kationischen, nichtionischen oder amphoteren Tensiden ausgewählt ist.

37. Zusammensetzung nach Anspruch 36, **dadurch gekennzeichnet, dass** der oder die grenzflächenaktive(n) Stoff(e) nichtionisch sind.

38. Zusammensetzung nach den Ansprüchen 36 oder 37, **dadurch gekennzeichnet, dass** die grenzflächenaktiven Stoffe 0,01 bis 40 % des Gesamtgewichts der Zusammensetzung ausmachen.

39. Zusammensetzung nach einem der vorhergehenden Ansprüche, **dadurch gekennzeichnet, dass** sie ferner mindestens ein Reduktionsmittel in Mengenanteilen von 0,05 bis 3 Gew.-%, bezogen auf das Gesamtgewicht der Zusammensetzung, enthält.

40. Zusammensetzung nach einem der vorhergehenden Ansprüche, die gebrauchsfertig ist, **dadurch gekennzeichnet, dass** sie ferner ein Oxidationsmittel enthält.

41. Zusammensetzung nach Anspruch 40, **dadurch gekennzeichnet, dass** sie einen pH-Wert von 4 bis 11 aufweist.

42. Zusammensetzung nach Anspruch 40, **dadurch gekennzeichnet, dass** das Oxidationsmittel unter Wasserstoffperoxid, Harnstoffperoxid, Bromaten oder Ferricyaniden von Alkalimetallen, Salzen von Persäuren und Redox-Enzymen gegebenenfalls zusammen mit ihrem jeweiligen Donor oder Cofaktor ausgewählt ist.

43. Zusammensetzung nach Anspruch 42, **dadurch gekennzeichnet, dass** das Oxidationsmittel Wasserstoffperoxid ist.

44. Zusammensetzung nach Anspruch 43, **dadurch gekennzeichnet, dass** das Oxidationsmittel eine Wasserstoffperoxidlösung ist, deren Titer im Bereich von 1 bis 40 Volumina liegt.

45. Verfahren zum Färben von Keratinfasern und insbesondere menschlichen Keratinfasern, wie Haaren, **dadurch gekennzeichnet, dass** es darin besteht, auf die Fasern mindestens eine färbende Zusammensetzung aufzutragen, die in einem zum Färben geeigneten Medium mindestens ein Farbstoffvorprodukt eines Oxidationsfarbstoffes und/oder einen Kuppler enthält, die unter den p-Phenylendiaminen, Doppelbasen, p-Aminophenolen, m-Phenylendiaminen, m-Aminophenolen, m-Dihydroxybenzolen, heterocyclischen Basen und heterocyclischen Kupplern und den Additionssalzen dieser Verbindungen mit einer Säure ausgewählt sind, wobei die Farbe bei einem alkalischen, neutralen oder sauren pH-Wert mit einer oxidierenden Zusammensetzung entwickelt wird, die mindestens ein Oxidationsmittel enthält und die bei der Anwendung mit der färbenden Zusammensetzung vermischt wird oder die getrennt davon ohne zwischenzeitliches Spülen aufgetragen wird, wobei mindestens ein amphoteres Polymer, das mindestens eine Fettkette aufweist, nach einem der Ansprüche 1 bis 10 in der färbenden Zusammensetzung oder in der oxidierenden Zusammensetzung oder in beiden Zusammensetzungen enthalten ist.

46. Verfahren nach Anspruch 30, **dadurch gekennzeichnet, dass** es darin besteht, auf die trockenen oder feuchten Keratinfasern die gebrauchsfertige Zusammensetzung, die bedarfsgemäß bei der Anwendung aus der färbenden Zusammensetzung und der oxidierenden Zusammensetzung hergestellt wird, aufzubringen, während einer Zeitspanne von etwa 1 bis 60 Minuten und vorzugsweise 5 bis 45 Minuten einwirken zu lassen, die Fasern zu spülen, gegebenenfalls mit Haarwaschmittel zu waschen, nochmals zu spülen und zu trocknen.

47. Vorrichtung mit mehrere Abteilungen oder "Kit" zum Färben von Keratinfasern und insbesondere menschlichen Keratinfasern, wie Haaren, **dadurch gekennzeichnet, dass** sie mindestens zwei Abteilungen enthält, wobei eine Abteilung eine färbende Zusammensetzung enthält, die in einem zum Färben geeigneten Medium mindestens ein Farbstoffvorprodukt eines Oxidationsfarbstoffes und/oder einen Kuppler enthält, die unter den p-Phenylendiaminen, Doppelbasen, p-Aminophenolen, m-Phenylendiaminen, m-Aminophenolen, m-Dihydroxybenzolen, heterocyclischen Basen und heterocyclischen Kupplern und den Additionssalzen dieser Verbindungen mit einer Säure ausgewählt sind, und eine andere Abteilung eine oxidierende Zusammensetzung enthält, die in einem zum Färben geeigneten Medium ein Oxidationsmittel enthält, wobei ein amphoteres Polymer, das mindestens eine Fettkette aufweist, nach einem der Ansprüche 1 bis 10 in der färbenden Zusammensetzung oder der oxidierenden Zusammensetzung oder in beiden Zusammensetzungen enthalten ist.

## Claims

1. Composition for the oxidation dyeing of keratin fibres, particularly human keratin fibres such as hair, comprising, in an appropriate dyeing medium, at least one oxidation dyestuff precursor and/or coupler selected from paraphenylenediamines, double bases, paraaminophenols, metaphenylenediamines, metaaminophenols, metadiphenols, bases and heterocyclic couplers, and the addition salts of these compounds with an acid, and at least one amphoteric polymer with at least one fatty chain having from 8 to 30 carbon atoms.

2. Composition according to Claim 1, **characterized in that** the amphoteric polymer with at least one fatty chain also contains at least one non-cyclic cationic unit.

3. Composition according to Claim 1 or 2, **characterized in that** said amphoteric polymer comprises 1 to 20 mol% of monomer with a fatty chain, based on the total number of moles of monomers.

4. Composition according to any one of Claims 1 to 3, **characterized in that** the amphoteric polymers comprise:
1) at least one monomer of formula (Ia) or (Ib) below: 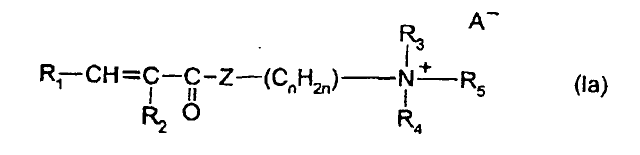 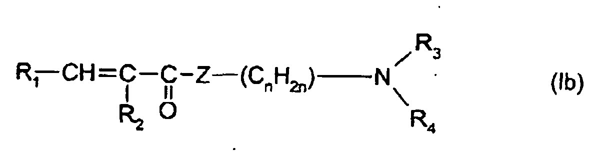 in which R₁ and R₂, which are identical or different, are a hydrogen atom or a methyl radical, R₃, R₄ and R₅, which are identical or different, are a linear or branched alkyl radical having from 1 to 30 carbon atoms,
Z is an NH group or an oxygen atom,
n is an integer from 2 to 5,
A⁻ is an anion derived from an organic or mineral acid;
2) at least one monomer of formula (II)
**R**_{**6**}**-CH=CR**_{**7**} **-COOH (II)** (II)
in which R₆ and R₇, which are identical or different, are a hydrogen atom or a methyl radical; and
3) at least one monomer of formula (III):
**R**_{**6**}**-CH=CR**_{**7**} **-COXR**_{**8**} **(III)** (III)
in which R₆ and R₇, which are identical or different, are a hydrogen atom or a methyl radical, X denotes an oxygen or nitrogen atom and R₈ denotes a linear or branched alkyl radical having from 1 to 30 carbon atoms;
at least one of the monomers of formula (Ia), (Ib) or (III) containing at least one fatty chain having from 8 to 30 carbon atoms;

5. Composition according to Claim 4, **characterized in that** the monomers of formulae (Ia) and (Ib) are selected from the group comprising dimethylaminoethyl methacrylate, dimethylaminoethyl acrylate, diethylaminoethyl methacrylate, diethylaminoethyl acrylate, dimethylaminopropyl methacrylate, dimethylaminopropyl acrylate, dimethylaminopropylmethacrylamide and dimethylaminopropylacrylamide, which are optionally quaternized.

6. Composition according to either of Claims 4 and 5, **characterized in that** the monomer of formula (Ia) is acrylamidopropyltrimethylammonium chloride or methacrylamidopropyltrimethylammonium chloride.

7. Composition according to Claim 4, **characterized in that** the monomer of formula (II) is selected from the group comprising acrylic acid, methacrylic acid, crotonic acid and 2-methylcrotonic acid.

8. Composition according to Claim 4, **characterized in that** the monomer of formula (III) is selected from the group comprising C₁₂-C₂₂ and preferably C₁₆-C₁₈ alkyl acrylates or methacrylates.

9. Composition according to any one of Claims 1 to 8, **characterized in that** said amphoteric polymers with a fatty chain are selected from acrylic acid/(meth)acrylamidopropyltrimethylammonium chloride/stearyl methacrylate terpolymers.

10. Composition according to Claim 1, **characterized in that** the amphoteric polymers with a fatty chain are selected from polymers derived from polyaspartic acid.

11. Composition according to any one of the preceding claims, **characterized in that** the amphoteric polymer with a fatty chain is used in an amount of between 0.05 and 10% by weight, based on the total weight of the composition.

12. Composition according to Claim 11, **characterized in that** the amphoteric polymer with a fatty chain is used in an amount of between 0.1 and 5% by weight, based on the total weight of the composition.

13. Composition according to Claim 1, **characterized in that** the paraphenylenediamines are selected from those of formula (I) below and their acid addition salts: 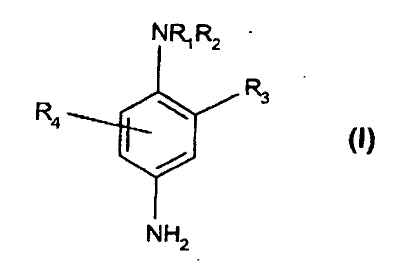 in which:
R₁ is a hydrogen atom, a C₁-C₄ alkyl radical, a C₁-C₄ monohydroxyalkyl radical, a C₂-C₄ polyhydroxyalkyl radical, an alkoxy (C₁-C₄) alkyl (C₁-C₄) radical or a C₁-C₄ alkyl radical substituted by a nitrogen, phenyl or 4'-aminophenyl group;
R₂ is a hydrogen atom, a C₁-C₄ alkyl radical, a C₁-C₄ monohydroxyalkyl radical, a C₂-C₄ polyhydroxyalkyl radical, an alkoxy (C₁-C₉) alkyl (C₁-C₄) radical or a C₁-C₄ alkyl radical substituted by a nitrogen group;
R₃ is a hydrogen atom, a halogen atom such as a chlorine atom, or a C₁-C₄ alkyl, sulpho, carboxyl, C₁-C₄ monohydroxyalkyl, C₁-C₄ hydroxyalkoxy, C₁-C₄ acetylaminoalkoxy, C₁-C₄ mesylaminoalkoxy or C₁-C₄ carbamoylaminoalkoxy radical; and
R₄ is a hydrogen or halogen atom or a C₁-C₄ alkyl radical,
it also being possible for R₁ and R₂ to form, with the nitrogen atom which carries them, a 5- or 6-membered nitrogen heterocycle optionally substituted by one or more alkyl, hydroxyl or ureido groups.

14. Composition according to Claim 13, **characterized in that** the nitrogen radicals are selected from amino, monoalkyl (C₁-C₄) amino, dialkyl (C₁-C₄) amino, trialkyl (C₁-C₄) amino, monohydroxyalkyl (C₁-C₄) amino, imidazolinium and ammonium radicals.

15. Composition according to either of Claims 13 and 14, **characterized in that** the paraphenylenediamines are selected from paraphenylenediamine, paratoluylenediamine, 2-chloro-paraphenylenediamine, 2,3-dimethylparaphenylenediamine, 2,6-dimethylparaphenylenediamine, 2,6-diethylparaphenylenediamine, 2,5-dimethylparaphenylenediamine, N,N-dimethylparaphenylenediamine, N,N-diethylparaphenylenediamine, N,N-dipropylparaphenylenediamine, 4-amino-N,N-diethyl-3-methylaniline, N,N-bis(β-hydroxyethyl)paraphenylenediamine, 4-N,N-bis(β-hydroxyethyl) amino-2-methylaniline, 4-N,N-bis(β-hydroxyethyl) amino-2-chloroaniline, 2-β-hydroxyethylparaphenylenediamine, 2-fluoroparaphenylenediamine, 2-isopropylparaphenylenediamine, N-(β-hydroxypropyl) paraphenylenediamine, 2-hydroxymethylparaphenylenediamine, N,N-dimethyl-3-methylparaphenylenediamine, N,N-(ethyl, β-hydroxyethyl) paraphenylenediamine, N-(β,γ-dihydroxypropyl) paraphenylenediamine, N-(4'-aminophenyl)paraphenylenediamine, N-phenyl-paraphenylenediamine, 2-β-hydroxyethyloxyparaphenylenediamine, 2-β-acetylaminoethyloxyparaphenylenediamine, N-(β-methoxyethyl)paraphenylenediamine, 2-methyl-1-N-β-hydroxyethylparaphenylenediamine and their acid addition salts.

16. Composition according to Claim 1, **characterized in that** the double bases are selected from those of formula (II) below and their acid addition salts: 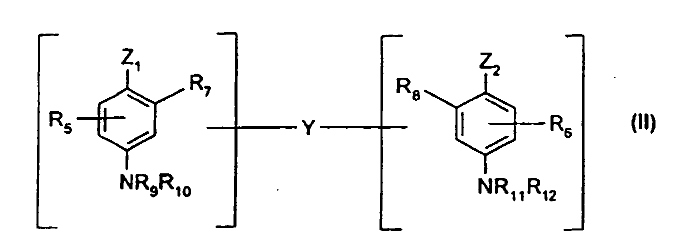 in which:
- Z₁ and Z₂, which are identical or different, are a hydroxyl or -NH₂ radical which can be substituted by a C₁-C₄ alkyl radical or by a linking arm Y;
- the linking arm Y is a linear or branched alkylene chain containing from 1 to 14 carbon atoms which can be interrupted or terminated by one or more nitrogen groups and/or by one or more heteroatoms such as oxygen, sulphur or nitrogen atoms, and optionally substituted by one or more hydroxyl or C₁-C₆ alkoxy radicals;
- R₅ and R₆ are a hydrogen or halogen atom, a C₁-C₄ alkyl, C₁-C₄ monohydroxyalkyl, C₂-C₄ polyhydroxyalkyl or C₁-C₄ aminoalkyl radical or a linking arm Y; and
- R₇, R₈, R₉, R₁₀, R₁₁ and R₁₂, which are identical or different, are a hydrogen atom, a linking arm Y or a C₁-C₄ alkyl radical,
it being understood that the compounds of formula (II) contain only one linking arm Y per molecule.

17. Composition according to Claim 16, **characterized in that**, in formula (II), the nitrogen groups are selected from amino, monoalkyl(C₁-C₄)amino, dialkyl (C₁-C₄) amino, trialkyl (C₁-C₄) amino, monohydroxyalkyl(C₁-C₄)amino, imidazolinium and ammonium radicals.

18. Composition according to either of Claims 16 and 17, **characterized in that** the double bases are selected from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)-ethylenediamine, N,N'-bis(4-aminophenyl)-tetramethylenediamine, N,N'-bis(β-hydroxyethyl)-N,N'-bis(4-aminophenyl)tetramethylenediamine, N, N'-bis(4-methylaminophenyl)tetramethylenediamine, N,N'-bis(ethyl)-N,N'-bis(4'-amino,3'-methylphenyl)-ethylenediamine, 1,8-bis(2,5-diaminophenoxy)-3,5-dioxaoctane and their acid addition salts.

19. Composition according to Claim 1, **characterized in that** the paraaminophenols are selected from those of formula (III) below and their acid addition salts: 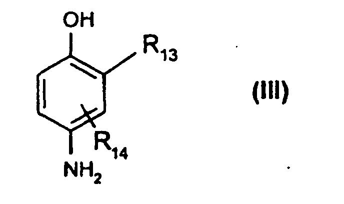 in which:
R₁₃ is a hydrogen atom, a halogen atom such as fluorine, or a C₁-C₄ alkyl, C₁-C₄ monohydroxyalkyl, alkoxy (C₁-C₄) alkyl (C₁-C₄), C₁-C₄ aminoalkyl or hydroxyalkyl (C₁-C₄) aminoalkyl (C₁-C₄) radical; and
R₁₄ is a hydrogen atom, a halogen atom such as fluorine, or a C₁-C₄ alkyl, C₁-C₄ monohydroxyalkyl, C₂-C₄ polyhydroxyalkyl, C₁-C₄ aminoalkyl, C₁-C₄ cyanoalkyl or alkoxy(C₁-C₄) alkyl (C₁-C₄) radical.

20. Composition according to Claim 19, **characterized in that** the paraaminophenols are selected from 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and their acid addition salts.

21. Composition according to Claim 1, **characterized in that** the heterocyclic bases are selected from pyridine derivatives, pyrimidine and pyrazolopyrimidine derivatives, pyrazole derivatives and their acid addition salts.

22. Composition according to Claim 21, **characterized in that** the pyridine derivatives are selected from 2,5-diaminopyridine, 2-(9-methoxyphenyl)amino-3-aminopyridine, 2,3-diamino-6-methoxypyridine, 2-(β-methoxyethyl)amino-3-amino-6-methoxypyridine, 3,4-diaminopyridine and their acid addition salts.

23. Composition according to Claim 21, **characterized in that** the pyrimidine derivatives are selected from 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine and 2,5,6-triaminopyrimidine, and the pyrazolopyrimidine derivatives are selected from pyrazolo[1,5-a]pyrimidine-3,7-diamine, 2,5-dimethylpyrazolo[1,5-a]pyrimidine-3,7-diamine, pyrazolo[1,5-a]pyrimidine-3,5-diamine, 2,7-dimethylpyrazolo[1,5-a]pyrimidine-3,5-diamine, 3-aminopyrazolo[1,5-a]pyrimidin-7-ol, 3-aminopyrazolo[1,5-a]pyrimidin-5-ol, 2-(3-aminopyrazolo[1,5-a]pyrimidin-7-ylamino)ethanol, 2-(7-aminopyrazolo[1,5-a]pyrimidin-3-ylamino)ethanol, 2-[(3-aminopyrazolo[1,5-a]pyrimidin-7-yl)(2-hydroxyethyl)amino]ethanol, 2-[(7-aminopyrazolo-[1,5-a]pyrimidin-3-yl)(2-hydroxyethyl)-amino]ethanol, 5,6-dimethylpyrazolo[1,5-a]pyrimidine-3,7-diamine, 2,6-dimethylpyrazolo[1,5-a]pyrimidine-3,7-diamine, 2,5, N7,N7-tetramethylpyrazolo[1,5-a]pyrimidine-3,7-diamine, 3-amino-5-methyl-7-imidazolylpropylaminopyrazolo-[1,5-a]pyrimidine and their addition salts, and their tautomeric forms if there is a tautomeric equilibrium, and their acid addition salts.

24. Composition according to Claim 21, **characterized in that** the pyrazole derivatives are selected from 4,5-diamino-1-methylpyrazole, 3,4-diaminopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-tert-butyl-1-methylpyrazole, 4,5-diamino-1-tert-butyl-3-methylpyrazole, 4,5-diamino-1-(β-hydroxyethyl)-3-methylpyrazole, 4,5-diamino-1-(β-hydroxyethyl)pyrazole, 4,5-diamino-1-ethyl-3-methylpyrazole, 4,5-diamino-1-ethyl-3-(4'-methoxyphenyl)pyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2'-aminoethyl)amino-1,3-dimethylpyrazole, 3,4,5-triaminopyrazole, 1-methyl-3,4,5-triaminopyrazole, 3,5-diamino-1-methyl-4-methylaminopyrazole, 3,5-diamino-4-(β-hydroxyethyl)amino-1-methylpyrazole and their acid addition salts.

25. Composition according to any one of the preceding claims, **characterized in that** the oxidation dyestuff precursors are present in concentrations ranging from 0.0005 to 12% by weight, based on the total weight of the composition.

26. Composition according to Claim 1, **characterized in that** the couplers are selected from metaphenylenediamines, metaaminophenols, metadiphenols, heterocyclic couplers and their acid addition salts.

27. Composition according to Claim 26, **characterized in that** the couplers are selected from 2,4-diamino-1-(β-hydroxyethyloxy)benzene, 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2-amino-4-(β-hydroxyethylamino)-1-methoxybenzene, 1,3-diaminobenzene, 1,3-bis(2,4-diaminophenoxy)propane, sesamol, 1-amino-2-methoxy-4,5-methylenedioxybenzene, 6-hydroxyindole, 4-hydroxyindole, 4-hydroxy-N-methylindole, 6-hydroxyindoline, 2,6-dihydroxy-4-methylpyridine, 1-H-3-methylpyrazol-5-one, 1-phenyl-3-methylpyrazol-5-one, 2-amino-3-hydroxypyridine, 3,6-dimethylpyrazolo[3,2-c]-1,2,4-triazole, 2,6-dimethylpyrazolo[1,5-b]-1,2,4-triazole and their acid addition salts.

28. Composition according to any one of the preceding claims, **characterized in that** the couplers are present in concentrations ranging from 0.0001 to 10% by weight, based on the total weight of the composition.

29. Composition according to any one of Claims 1 and 13 to 28, **characterized in that** the acid addition salts of the oxidation dyestuff precursors and of the couplers are selected from hydrochlorides, hydrobromides, sulphates, tartrates, lactates and acetates.

30. Composition according to any one of the preceding claims, **characterized in that** it also contains direct dyestuffs in a proportion of 0.001 to 20% by weight, based on the total weight of the composition.

31. Composition according to any one of the preceding claims, **characterized in that** it also contains at least one cationic polymer or at least one amphoteric polymer other than the amphoteric polymers with a fatty chain as defined in any one of Claims 1 to 10.

32. Composition according to Claim 31, **characterized in that** the cationic polymer is a quaternary polyammonium compound consisting of repeat units of formula (W) below:

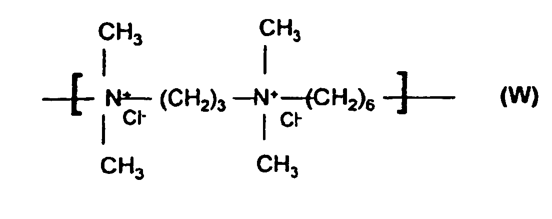

33. Composition according to Claim 31, **characterized in that** the cationic polymer is a quaternary polyammonium compound consisting of repeat units of formula (U) below:

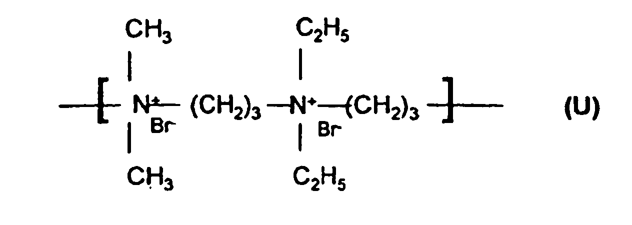

34. Composition according to Claim 31, **characterized in that** the amphoteric polymer is a copolymer comprising at least acrylic acid and a dimethyldiallylammonium chloride as monomers.

35. Composition according to any one of Claims 31 to 34, **characterized in that** the cationic polymer(s) or the amphoteric polymers other than those with a fatty chain according to the invention represent from 0.01% to 10% by weight, based on the total weight of the composition.

36. Composition according to any one of the preceding claims, **characterized in that** it contains at least one surfactant selected from anionic, cationic, non-ionic or amphoteric surfactants.

37. Composition according to Claim 36, **characterized in that** the surfactant(s) is(are) non-ionic.

38. Composition according to Claim 36 or 37, **characterized in that** the surfactants represent 0.01 to 40% by weight, based on the total weight of the composition.

39. Composition according to any one of the preceding claims, **characterized in that** it also contains at least one reducing agent in amounts ranging from 0.05 to 3% by weight, based on the total weight of the composition.

40. Ready-to-use composition according to any one of the preceding claims, **characterized in that** it also contains an oxidizing agent.

41. Composition according to Claim 40, **characterized in that** it has a pH ranging from 4 to 11.

42. Composition according to Claim 40, **characterized in that** the oxidizing agent is selected from hydrogen peroxide, urea peroxide, alkali metal bromates or ferricyanides, persalts and redox enzymes, optionally with their respective donor or cofactor.

43. Composition according to Claim 42, **characterized in that** the oxidizing agent is hydrogen peroxide.

44. Composition according to Claim 43, **characterized in that** the oxidizing agent is a hydrogen peroxide solution whose strength varies from 1 to 40 volumes.

45. Process for dyeing keratin fibres, particularly human keratin fibres such as hair, **characterized in that** it consists in applying to the fibres at least one dyeing composition containing, in an appropriate dyeing medium, at least one oxidation dyestuff precursor and/or coupler selected from paraphenylenediamines, double bases, paraaminophenols, metaphenylenediamines, metaaminophenols, metadiphenols, bases and heterocyclic couplers, and the addition salts of these compounds with an acid,
the colour being developed at alkaline, neutral or acidic pH with the aid of an oxidizing composition containing at least one oxidizing agent, which is mixed with the dyeing composition at the time of use or is applied sequentially without intermediate rinsing, at least one amphoteric polymer with at least one fatty chain, as defined according to Claims 1 to 10, being present in the dyeing composition, in the oxidizing composition or in each of said compositions.

46. Process according to Claim 30, **characterized in that** it consists in applying, to the dry or wet keratin fibres, the ready-to-use composition prepared at the time of use from the dyeing and oxidizing compositions, leaving it to act for a period of time varying from about 1 to 60 minutes, preferably from 5 to 45 minutes, rinsing the fibres, optionally shampooing them and then rinsing them again, and drying them.

47. Device with several compartments, or kit, for dyeing keratin fibres, particularly human keratin fibres such as hair, **characterized in that** it comprises at least two compartments, one of which comprises a dyeing composition containing, in an appropriate dyeing medium, at least one oxidation dyestuff precursor and/or coupler selected from paraphenylenediamines, double bases, paraaminophenols, metaphenylenediamines, metaaminophenols, metadiphenols, bases and heterocyclic couplers, and the addition salts of these compounds with an acid,
and another of which comprises an oxidizing composition containing, in an appropriate dyeing medium, an oxidizing agent, an amphoteric polymer with at least one fatty chain, as defined in any one of Claims 1 to 10, being present in the dyeing composition, in the oxidizing composition or in each of the two compositions.
